(19) Europäisches Patentamt
European Patent Office
Office européen des brevets

(11) Veröffentlichungsnummer: **0 221 844**
**A1**

# (12) EUROPÄISCHE PATENTANMELDUNG

(21) Anmeldenummer: **86810430.8**

(22) Anmeldetag: **01.10.86**

(51) Int. Cl.⁴: **C 07 D 213/30**
C 07 D 213/50, A 01 N 43/40

(30) Priorität: **01.10.85 CH 4245/85**

(43) Veröffentlichungstag der Anmeldung:
**13.05.87 Patentblatt 87/20**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Tobler, Hans, Dr.**
**Baselmattweg 157**
**CH-4123 Allschwil (CH)**

**Ackermann, Peter, Dr.**
**Hangelimattweg 113**
**CH-4148 Pfeffingen (CH)**

**Nyfeler, Robert, Dr.**
**Bärenfelserstrasse 8**
**CH-4057 Basel (CH)**

The title of the invention has been amended (Guidelines for Examination in the EPO, A-III, 7.3).

(54) **Pyridinderivate mit mikrobizider Wirkung.**

(57) Pyridinyldérivate der Formel I

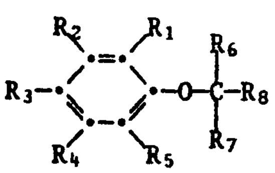

(I),

worin
$R_1$ bis $R_5$ unabhängig voneinander Wasserstoff, Halogen, $C_1$-$C_6$-Alkyl, $C_1$-$C_6$-Halogenalkyl, $C_1$-$C_6$-Alkoxy, $C_1$-$C_6$-Halogenalkoxy, Cyano, $C_1$-$C_6$-Alkoxycarbonyl oder Phenyl,
$R_6$ und $R_7$ unabhängig voneinander Wasserstoff, $C_1$-$C_6$-Alkyl, $C_3$-$C_6$-Alkenyl oder $C_3$-$C_6$-Alkinyl sowie gegebenenfalls durch Halogen, $C_1$-$C_6$-Alkyl, $C_1$-$C_6$-Halogenalkyl, $C_1$-$C_6$-Alkoxy oder $C_1$-$C_6$-Halogenalkoxy substituiertes Phenyl oder Benzyl,

$R_8$ 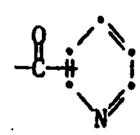 oder 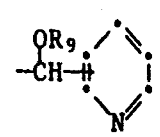 und

$R_9$ Wasserstoff, $C_1$-$C_6$-Alkyl, $C_3$-$C_6$-Alkenyl, $C_3$-$C_6$-Alkinyl oder gegebenenfalls durch Halogen, $C_1$-$C_6$-Alkyl, $C_1$-$C_6$-Halogenalkyl, $C_1$-$C_6$-Alkoxy, $C_1$-$C_6$-Halogenalkoxy substituiertes Benzyl oder einen Acylrest bedeuten.

Es werden Verfahren zur Herstellung dieser Verbindungen und ihre Verwendung in der Schädlingsbekämpfung, vor allem gegen Bakterien und Pilze beschrieben.

EP 0 221 844 A1

**Beschreibung**

Mikrobizide

Die vorliegende Erfindung betrifft Pyridinylderivate, Verfahren zu ihrer Herstellung und ihre Verwendung in der Schädlingsbekämpfung. Die Pyridinylderivate haben die Formel I

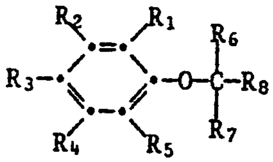 (I),

worin

$R_1$ bis $R_5$ unabhängig voneinander Wasserstoff, Halogen, $C_1$-$C_6$-Alkyl, $C_1$-$C_6$-Halogenalkyl, $C_1$-$C_6$-Alkoxy, $C_1$-$C_6$-Halogenalkoxy, Cyano, $C_1$-$C_6$-Alkoxycarbonyl oder Phenyl,

$R_6$ und $R_7$ unabhängig voneinander Wasserstoff, $C_1$-$C_6$-Alkyl, $C_3$-$C_6$-Alkenyl oder $C_3$-$C_6$-Alkinyl sowie Phenyl oder Benzyl, deren aromatischer Ring unsubstituiert oder durch Halogen, $C_1$-$C_6$-Alkyl, $C_1$-$C_6$-Halogenalkyl, $C_1$-$C_6$-Alkoxy oder $C_1$-$C_6$-Halogenalkoxy substituiert sein kann,

$R_8$ 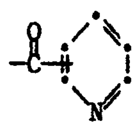 oder 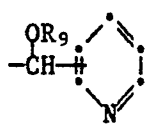 und

$R_9$ Wasserstoff, $C_1$-$C_6$-Alkyl, $C_3$-$C_6$-Alkenyl, $C_3$-$C_6$-Alkinyl oder Benzyl, das unsubstituiert oder im Ring durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert sein kann, bedeuten mit der Massgabe, dass eine CO-Gruppe im Substituenten $R_8$ in 3- oder 4-Stellung des Pyridinrings steht, falls $R_1$, $R_2$, $R_4$, $R_5$ and $R_7$ gleichzeitig Wasserstoff, $R_3$ Methoxy und $R_6$ Methyl bedeuten und, wobei ferner

einen Acylrest $R_{10}$-CO- bedeuten kann, in dem $R_{10}$ $C_1$-$C_6$-Alkyl, $C_3$-$C_6$-Alkenyl, $C_3$-$C_6$-Alkinyl, $C_2$-$C_5$-Alkoxyalkyl oder $C_1$-$C_6$-Haloalkyl oder einen $C_3$-$C_6$-Cycloalkylrest bedeutet, der unsubstituiert oder durch $C_1$-$C_3$-Alkyl substituiert sein kann, oder einen der Reste Phenyl, Benzyl, Phenethyl darstellt, die unsubstituiert oder im Ring durch Halogen, $C_1$-$C_6$Alkyl, $C_1$-$C_6$Halogenalkyl, $C_1$-$C_6$Alkoxy, $C_1$-$C_6$Halogenalkoxy substituiert sein können.

Die bei $R_1$ bis $R_{10}$ in Frage kommenden Alkyl-, Halogenalkyl-, Alkoxy-, Halogenalkoxy-, Alkenyl- und Alkinylgruppen können geradkettig oder verzweigt sein.

Unter dem Begriff Alkyl selbst oder als Bestandteil eines anderen Substituenten wie Alkoxy, Halogenalkyl, Halogenalkoxy etc., sind je nach Zahl der angegebenen Kohlenstoffatome beispielsweise die folgenden Gruppen zu verstehen: Methyl, Aethyl, Propyl, Butyl, Pentyl, Hexyl sowie ihre Isomeren, wie z.B. Isopropyl, Isobutyl, tert.-Butyl, Isopentyl usw. Halogen und Halo stehen stellvertretend für Fluor, Chlor, Brom oder Jod. Haloalkyl bzw. Halogenalkyl steht somit für einen einfach bis perhalogenierten Alkylrest, wie z.B. $CHCl_2$, $CH_2F$, $CCl_3$, $CH_2Cl$; $CHFCH_3$ $CH_2CH_2Br$, $CF_2CF_3$, $C_2Cl_5$, $CH_2Br$, CHBrCl usw., vorzugsweise für $CF_3$ und $CHF_2$. Die vorstehenden Beispiele gelten sinngemäss für Halogenalkoxy. Alkenyl steht z.B. für Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl. Als Beispiele für $C_2$-$C_5$-Alkoxyalkyl seien Propoxyäthyl, Aethoxyäthyl, Aethoxymethyl, Methoxymethyl genannt.

Eine Gruppe von Verbindungen sind jene der Formel I, worin $R_1$ bis $R_9$ die genannte Bedeutung haben, mit Ausnahme der Definition $R_{10}$CO-für den Substituenten $R_9$.

Bevorzugt sind Verbindungen der Formel I, worin

$R_1$ bis $R_5$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy sind, oder einer dieser Substituenten auch Phenyl sein kann,

$R_6$ und $R_7$ unabhängig voneinander Wasserstoff, $C_1$-$C_6$-Alkyl, $C_3$-$C_5$-Alkenyl, $C_3$-$C_5$-Alkinyl, Phenyl oder Benzyl bedeuten, die im Ring durch Halogen substituiert sein können, und

$R_9$ Wasserstoff, $C_1$-$C_4$-Alkyl, $C_3$-$C_4$-Alkenyl, $C_3$-$C_4$-Alkinyl, Benzyl oder Halobenzyl darstellt, oder aber die Bedeutung $R_{10}$CO- hat, in der $R_{10}$ $C_1$-$C_4$-Alkyl, $C_3$-$C_4$-Alkenyl, $C_3$-$C_4$-Alkinyl, $C_2$-$C_5$-Alkoxyalkyl, $C_1$-$C_4$-Haloalkyl oder einen $C_3$-$C_6$-Cycloalkylrest bedeutet, der unsubstituiert oder durch $C_1$-$C_3$-Alkyl substituiert ist, oder einen der Reste Phenyl oder Benzyl darstellt, die unsubstituiert oder maximal zweifach durch Halogen, $C_1$-$C_2$-Alkyl, $C_1$-$C_2$-Alkoxy oder $C_1$-$C_2$-Halogenalkyl, das 1-3 Halogenatome enthält, substituiert sein können, während $R_8$ die für Formel I angegebenen Bedeutung hat.

Unter diesen letztgenannten bilden solche Verbindungen der Formel I eine Gruppe, worin $R_1$ bis $R_8$ die vorgenannte Bedeutung haben, und

$R_9$ Wasserstoff, $C_1$-$C_4$-Alkyl, $C_3$-$C_4$-Alkenyl, $C_3$-$C_4$-Alkinyl, Benzyl oder Halobenzyl darstellt, während $R_8$ die für Formel I angegebene Bedeutung hat. (Verbindungsgruppe Ia).

Eine der wichtigen Untergruppen sind jene Pyridinyl-Derivate der Formel I mit $R_8$ = CO-Pyridinyl(3) oder -CH($OR_9$)-Pyridinyl(3), worin
$R_1$ = Wasserstoff, Methyl, Aethyl oder Halogen,
$R_2$ = Wasserstoff,
$R_3$ = Wasserstoff, Halogen, $C_1$-$C_3$-Halogenalkyl, $C_1$-$C_3$-Halogenalkoxy, CN oder Phenyl,
$R_4$ = Wasserstoff oder Halogen,
$R_5$ = Wasserstoff oder Halogen,
$R_6$ = $C_1$-$C_4$-Alkyl, $C_3$-$C_5$-Alkenyl, $C_3$-$C_5$-Alkinyl, Phenyl oder Benzyl darstellt, die im Ring durch Halogen substituiert sein können,
$R_7$ = Wasserstoff oder $C_1$-$C_3$-Alkyl, und
$R_9$ = Wasserstoff, $C_1$-$C_4$-Alkyl, Allyl, Propargyl, Benzyl oder
$R_{10}$CO-bedeutet, wobei $R_{10}$ $C_1$-$C_4$-Alkyl, $C_3$-$C_4$-Alkenyl, $C_1$-$C_4$-Haloalkyl oder $C_3$-$C_6$-Cycloalkyl ist, das unsubstituiert oder durch Methyl oder Aethyl substituiert ist.

Eine weitere wichtige Untergruppe sind jene Pyridinyl(3)-Derivate der Formel I, deren Substituenten $R_1$ bis $R_8$ so wie die vorstehend genannten substituiert sind, worin $R_6$ zusätzlich Wasserstoff ist, und $R_9$ Wasserstoff, $C_1$-$C_4$-Alkyl, Allyl, Propargyl oder Benzyl darstellt. (Verbindungsgruppe I-b).

Innerhalb der Verbindungsgruppe I-b sind jene Verbindungen bevorzugt, worin
$R_1$ = Wasserstoff, Fluor oder Chlor,
$R_3$ = Fluor, Chlor oder Brom, $CF_3$, $CF_3$-O- oder $CHF_2$-O-, und $R_2$,
$R_4$ und $R_5$ Wasserstoff bedeuten, $R_6$ Wasserstoff, $C_2$-$C_4$-Alkyl, Allyl, Propargyl, Phenyl oder Chlorphenyl, $R_7$ Wasserstoff oder Methyl und
$R_9$ = Wasserstoff, Methyl, Aethyl, Isopropyl, Allyl oder Benzyl darstellen. (Verbindungsgruppe I-c). In der Verbindungsgruppe I-c sind jene Vertreter besonders bevorzugt, worin $R_6$ für einen der genannten aliphatischen Substituenten steht.

Eine weitere wichtige Untergruppe der Formel I sind Verbindungen, worin
$R_8$ CO-Pyridinyl(3) oder CH($OR_9$)-Pyridinyl(3) darstellt,
$R_1$ = Wasserstoff, Methyl, Fluor oder Chlor,
$R_3$ = Fluor, Chlor oder Brom, $CF_3$, $CF_3$-O- oder $CHF_2$-O-, und $R_2$, $R_4$ und $R_5$ Wasserstoff bedeuten, $R_6$ $C_2$-$C_4$-Alkyl, Allyl, Propargyl, Phenyl oder Chlorphenyl ist,
$R_7$ = Wasserstoff oder Methyl, und
$R_9$ die Gruppe $R_{10}$CO- bedeutet, in der $R_{10}$ für $C_1$-$C_4$-Alkyl, $C_3$-$C_4$-Alkenyl, $C_1$-$C_2$-Haloalkyl oder $C_3$-$C_5$-Cycloalkyl steht, das unsubstituiert oder durch Methyl oder Aethyl substituiert ist.

Eine bevorzugte Untergruppe von Verbindungen der Formel I sind solche, worin $R_1$ und $R_3$ Chlor und $R_2$, $R_4$, $R_5$ und $R_7$ Wasserstoff bedeuten, $R_6$ $C_1$-$C_4$-Alkyl ist, und $R_8$ CO-Pyridinyl(3) oder CH($OR_9$)-Pyridinyl(3) darstellt, wobei $R_9$ Wasserstoff oder $R_{10}$CO- bedeutet und $R_{10}$ $C_1$-$C_4$-Alkyl, $C_2$-$C_5$-Alkoxyalkyl, $C_3$-$C_4$-Alkenyl, $CF_3$, Cyclopropyl oder Methylcyclopropyl ist.

Unter den letztgenannten Verbindungen sind solche besonders bevorzugt, worin $R_{10}$ Methyl, Aethyl, Propyl, Isopropyl, Cyclopropyl, Butyl, sek. Butyl, Isobutyl oder tert. Butyl darstellt, während $R_1$ bis $R_8$ die angegebenen Bedeutungen haben.

Unter den bevorzugten Beispielen von Verbindungen der Formel I sind u.a. folgender Vertreter:

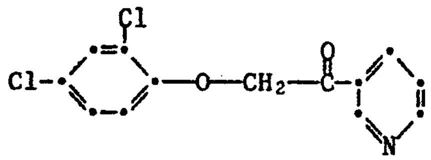

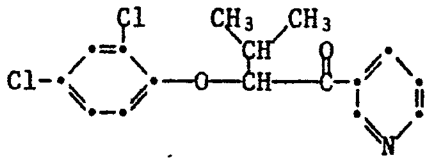

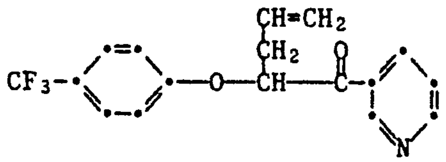

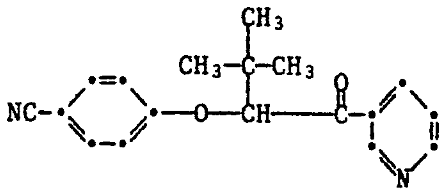

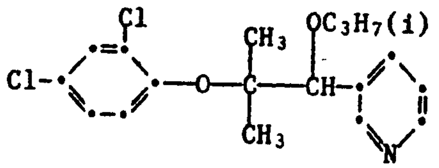

,

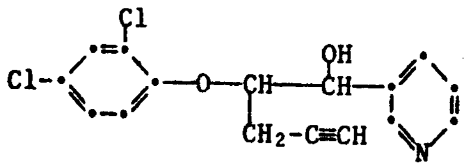

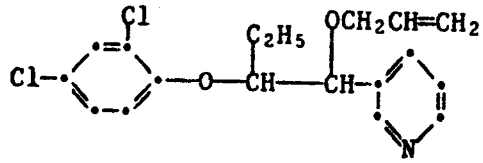

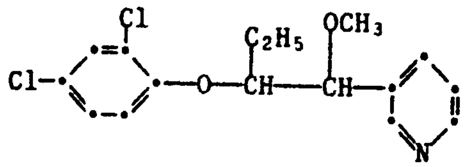

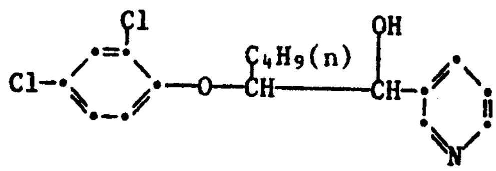

sowie die Verbindungen
1-(3-Pyridinyl)-2-(2,4-dichlorphenoxy)-butan-1-on,
1-(3-Pyridinyl)-2-(2,4-dichlorphenoxy)-butan-1-ol,
1-(3-Pyridinyl)-1-acetoxy-2-(2,4-dichlorphenoxy)-butan,
1-(3-Pyridinyl)-1-propionyloxy-2-(2,4-dichlorphenoxy)-butan,
1-(3-Pyridinyl)-1-methoxyacetoxy-2-(2,4-dichlorphenoxy)-butan,
1-(3-Pyridinyl)-2-(4-chlorphenoxy)-hexan-1-on,
1-(3-Pyridinyl)-2-(2,4-dichlorphenoxy)-pentan-1-ol.

Die erfindungsgemässen Verbindungen der Formel I, worin $R_8$

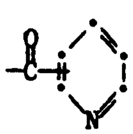

bedeutet, können z.B. erhalten werden, indem man

a) einen Ester der Formel II

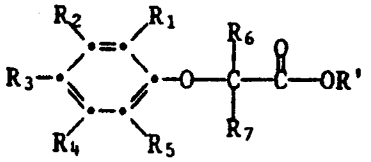

(II)

mit einem Halogenid der Formel III

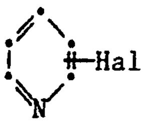

(III)

in Gegenwart eines Metallierungsmittels wie Mg (z.B. in Form einer Grignard-Verbindung) oder wie Butyllithium umsetzt oder

b) eine Verbindung der Formel IV

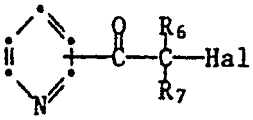

(IV)

mit einem Phenol der Formel V

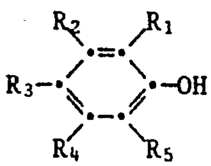

(V)

in Gegenwart einer Base reagieren lässt, wobei $R_1$ bis $R_7$ die für Formel I gegebene Bedeutung haben und Hal Halogen, bevorzugt Chlor oder Brom, bedeutet, während R' für $C_1$-$C_4$-Alkyl, $C_3$-$C_4$-Alkenyl oder für Phenyl oder Benzyl steht, die unsubstituiert oder durch Alkyl, Alkoxy, Halogen, $NO_2$, CN ringsubstituiert sein können.

Als Basen kommen bei dem Verfahren b) insbesondere tertiäre Amine, wie Trialkylamine und Pyridin, ferner Hydroxide, Oxide, Carbonate und Bicarbonate von Alkali- und Erdalkalimetallen sowie Alkalimetallalkoholate, wie z.B. Kalium-t.butylat oder Alkalihydride in Betracht.

Sollen Verbindungen der Formel I hergestellt werden, bei denen $R_6$ und/oder $R_7$ eine andere Bedeutung als Wasserstoff haben, so ist es zuweilen vorteilhaft, in einem ersten Reaktionsschritt eine Verbindung der Formel IV, bei der $R_6 = R_7 =$ Wasserstoff bedeutet, mit dem gewünschten Phenol der Formel V zur Reaktion zu bringen, und dann in einem Folgeschritt in die entstandene Verbindung der Formel I den gewünschten Substituenten $R_6$ und/oder $R_7$ an der aktivierten $CH_2$-Gruppe in Form einer Verbindung

$R_6$-hal (VIa) oder $R_7$-hal (VIb)

einzuführen, wobei hal Halogen, bevorzugt Brom oder Jod, bedeutet. Diese Substitution wird in Gegenwart starker Basen wie Na-Methylat oder bevorzugt K-tert.Butylat durchgeführt.

Das Verfahren a) wird entweder in Abwesenheit oder bevorzugt in Anwesenheit eines Lösungs- oder Verdünnungsmittels und bei einer Temperatur im Bereich von -130° bis +50° C, vorzugsweise bei -130° bis + 20° C, durchgeführt.

Das Verfahren b) wird gleichfalls bevorzugt in einem Lösungsmittel bei Temperaturen von -50° bis +150° C, bevorzugt 0° bis 120° C, durchgeführt.

Als Lösungs- oder Verdünnungsmittel kommen inerte, hydroxylgruppenfreie Vertreter in Frage, z.B. Aether und ätherartige Verbindungen, wie Diäthyläther, Diisopropyläther, Dioxan, 1,2-Dimethoxyäthan und Tetrahydrofuran; Amide, wie N,N-dialkylierte Carbonsäureamide vom Typ Dimethylformamid; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton oder Methyläthylketon.

Die nach den oben stehenden Verfahren hergestellten Ketone der Formel I können nach bekannten Methoden durch Hydrierung und, sofern gewünscht, anschliessende Reaktion mit einer Verbindung der Formel VII

R"Hal (VII)

in Verbindungen der Formel I überführt werden,

worin $R_8$ 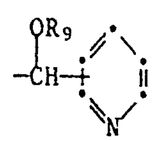

und $R_9$ Wasserstoff, $C_1$-$C_6$-Alkyl, $C_3$-$C_6$-Alkenyl, $C_3$-$C_6$-Alkinyl oder Benzyl, das im Ring durch Halogen,

$C_1$-$C_6$-Alkyl, $C_1$-$C_6$-Halogenalkyl, $C_1$-$C_6$-Alkoxy, $C_1$-$C_6$-Halogenalkoxy substituiert sein kann, bedeuten.

In der Formel VII steht R'' für $C_1$-$C_6$-Alkyl, $C_3$-$C_6$-Alkenyl, $C_3$-$C_6$-Alkinyl oder gegebenenfalls durch Halogen, $C_1$-$C_6$-Alkyl, $C_1$-$C_6$-Halogenalkyl, $C_1$-$C_6$-Alkoxy, $C_1$-$C_6$-Halogenalkoxy kernsubstituiertes Benzyl.

Verbindungen der Formel I, worin $R_9$ Wasserstoff bedeutet, lassen sich zur Erzielung von Acylverbindungen mit einer entsprechenden Carbonsäure $R_{10}$-COOH oder ihrem Säurehalogenid (besonders Säurechlorid oder -bromid) oder ihrem Säureanhydrid zur Reaktion bringen. Die Reaktionstemperatur liegt zweckmässig im Bereich vom -20° bis +80°C, und es können OH-freie Lösungs- oder Verdünnungsmittel verwendet werden (siehe oben).

Verbindungen der Formeln II bis VII sind bekannt oder können nach bekannten Verfahren hergestellt werden.

Verbindungen der Formel I können, falls sie ein asymmetrisches Kohlenstoffatom besitzen, in zwei enantiomeren Formen vorliegen. Im allgemeinen entsteht bei der Herstellung dieser Substanzen ein Gemisch beider Enantiomerer; dieses lässt sich auf bekannte Weise in die reinen optischen Antipoden aufspalten. Verbindungen der Formel I mit zwei Asymmetrie-Zentren liegen auch als Gemische von diastereomeren Formen vor, die mittels physikalischer Methoden getrennt werden können. Die vorliegende Erfindung betrifft alle reinen Enantiomeren bzw. Diastereomeren und deren Gemische untereinander.

Verbindungen der Formel I besitzen ein für praktische Bedürfnisse sehr günstiges Mikrobizid-Spektrum gegen phytopathogene Pilze und Bakterien. Sie haben sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe der Formel I sind gegen die den folgenden Klassen angehörenden phytopathogenen Pilze wirksam: Fungi imperfecti (z.B. Botrytis, Helminthosporium, Fusarium Septoria, Cercospora und Alternaria); Basidiomyceten (z.B. die Gattungen Hemileia, Rhizoctonia, Puccinia); insbesondere wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden. Die Verbindungen der Formel I besitzen teilweise auch pflanzenregulatorische Wirkung und können in höheren Dosierungen zur Zügelung des übermässigen vegetativen Wachstums an Kulturpflanzen verwendet werden.

Die Erfindung betrifft weiterhin mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindung der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Die strukturnächste bekannte Verbindung 2-(4-Methoxy-phenoxy)-1-(2-pyridinyl)-1-propanon wird in der DE-OS 29 09 754 als Zwischenprodukt zur Herstellung von pharmazeutisch wirkenden Benzofuran-Derivaten beschrieben. Eine mikrobizide Wirkung ist von ihr nicht bekannt geworden.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen,

Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwendmengen liegen im allgemeinen bei 50 g bis 5 kg Aktivsubstanz (AS) je ha, bevorzugt 100 g bis 2 kg AS/ha, insbesondere bei 150 g bis 600 g AS/ha.

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethyl-ether, Ketone, wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl; oder Wasser.

Als feste Trägerstoffe, z.B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nicht-sorptive Träger z.B. Calcit oder Dolomit in Frage. Es können auch zerkleinerte Pflanzenrückstände verwendet werden.

Als oberflächenaktive Verbindungen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood New Jersey, 1980
Sisley and Wood, "Encyclopedia of Surface Active Agents", Chemical Publishing Co., Inc. New York, 1980.
Besonders vorteilhafte, applikationsfördernde Zuschlagstoffe sind ferner natürliche oder synthetische Phospholipide aus der Reihe der Kephaline und Lecithine, wie z.B. Phosphatidylethanolamin, Phosphatidylserin, Phosphatidylglycerin, Lysolecithin.

Die agrochemischen Zubereitungen enthalten in der Regel 0,1 bis 99 %, insbesondere 0,1 bis 95 % Wirkstoff der Formel I, 99,9 bis 1 %, insbesondere 99,9 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellungsbeispiele für Wirkstoffe der Formel I

Beispiel 1.1: Herstellung von 2-(2,4-Dichlorphenoxy)-1-(3-pyridinyl)-1-äthanon

140,2 g 2,4-Dichlorphenol (93 %ig) und 232 g Kaliumcarbonat werden in 1000 ml Aceton vorgelegt. Nach kurzem Aufheizen auf Siedetemperatur wird auf 0°C gekühlt und innert einer Stunde portionenweise insgesamt 224,8 g 3-(Bromacetyl)-pyridin-hydrobromid eingetragen. Man rührt während 15 Stunden bei 0 bis 5°C und während 6 Stunden bei 20°C. Nach dem Filtrieren und Eindampfen des Reaktionsgemisches wird das Rohprodukt aus Methanol umkristallisiert.

Man erhält die Verbindung Nr. 1 der Formel

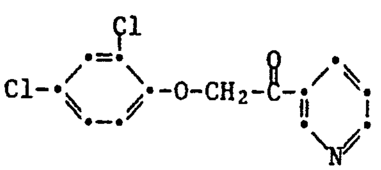

mit einem Schmelzpunkt von 118-119°C.

Beispiel 1.2: Herstellung von 2-(2,4-Dichlorphenoxy)-1-(3-pyridinyl)-1-propan-1-on

Zu 8.05 g Kalium-tert.-butylat in 140 ml trockenem Tetrahydrofuran werden unter Kühlung 17,38 g 2,4-Dichlorphenoxyacetyl-3-pyridin zugefügt, wobei die Innentemperatur auf 35°C steigt. In die Lösung tropft man bei 20°C 19,7 g Methyljodid und rührt bei 45°C während einer Stunde. Das filtrierte Rohprodukt wird säulenchromatographisch gereinigt (Kieselgel; Eluiermittel: Essigester/Hexan 1:1,5).

Man erhält die Verbindung Nr. 2 der Formel

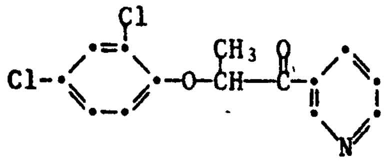

mit einer Refraktion von $n_D^{32°}$ = 1,5884.

Beispiel 1.3: Herstellung von 1-(3-Pyridinyl)-2-methyl-2-(4-chlorphenoxy)-propanon

12 ml Butyllithium in 1,6 ml Hexan werden in 50 ml Aether gelöst (Argonatmosphäre). Bei -70°C werden rasch 2,6 g 3-Brompyridin addiert. Nach 30 minütigem Rühren bei -70°C tropft man der Suspension 3,8 g 2-Methyl-2-(4-chlorphenoxy)-propionsäureäthylester zu. Nach 30 minütigem Rühren bei -70°C und einer Stunde bei 20°C wird das Reaktionsgemisch mit 20 ml gesättigter Ammoniumsulfatlösung sowie 5 ml gesättigter Sodalösung versetzt, getrocknet ($Na_2SO_4$), eingedampft und chromatographiert (Kieselgel: mit Essigester/Hexan 1:2 als Eluiermittel).

Man erhält die Verbindung Nr. 3 der Formel

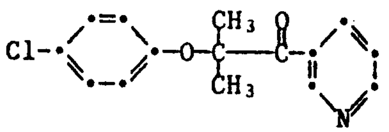

mit einem Schmelzpunkt von 44-47°C.

Beispiel 1.4: Herstellung von 1-(3-Pyridinyl)-2-(2,4-dichlorphenoxy)-butan-1-ol

11,16 g 2-(2,4-Dichlorphenoxy)-butyryl-3-pyridin in 105 ml Methanol werden portionenweise mit 1,8 g $NaBH_4$ versetzt. Nach 1,5 stündigem Rühren bei 40°C wird die Lösung eingedampft, in Essigester aufgenommen, mit Wasser und Sole gewaschen, getrocknet und eingedampft.

Das Rohprodukt kristallisiert aus Essigester/Hexan 1:1,5. Man erhält die Verbindung Nr. 4 der Formel

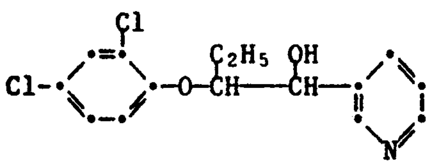

mit einem Schmelzpunkt von 118-120° (als im wesentlichen ein reines Diastereomeres).

Beispiel 1.5: Herstellung von 1-(3-Pyridinyl)-1-methoxy-2-(2,4-dichlorphenoxy)-butan

4,53 g 1-(3-Pyridyl)-2-(2,4-dichlorphenoxy)-butan-1-ol im wesentlichen reines Diastereomer in 15 ml Tetrahydrofuran werden zu einer Suspension von 0,8 g NaH (50%ige Oeldispersion; in Toluol gewaschen) in 14 ml Tetrahydrofuran getropft. Nach dem Aufhören der Wasserstoffentwicklung addiert man 10 ml Methyljodid und lässt während zwei Stunden bei 20°C aus reagieren. Das Reaktionsgemisch wird eingedampft, mit Wasser versetzt und mit Aether extrahiert. Die organische Phase wird abgetrennt, mit Wasser und Sole gewaschen, getrocknet ($Na_2SO_4$), gehärtet (Aktivkohle) und eingedampft. Nach dem Destillieren des Rohproduktes bei 145-150°C/$10^{-2}$ mbar erhält man die Verbindung Nr. 5 der Formel

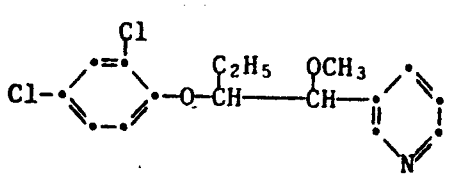

mit einer Refraktion von $n_D^{30°}$ = 1,5586 (als im wesentlichen ein reines diastereomeres).

Beispiel 1.6: Herstellung von 1-(3-Pyridinyl)-2-(2,4-dichlorphenoxy)-1-butanon der Formel

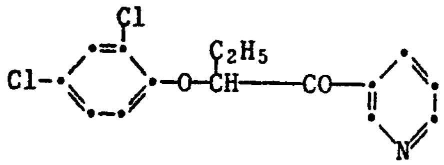

(Verb. Nr. 29)

Unter leichter Kühlung werden langsam 9.2 g 1-(3-Pyridyl)-2-(2,4-dichlorphenoxy)-1-äthanon zu einer Lösung von 4.15 g K-tert.Butylat in 45 ml Tetrahydrofuran gegeben. Zu der entstandenen dunkelroten Lösung tropft man 2.9 ml Aethyljodid, rührt 1 Stunde bei Raumtemperatur und 2 Stunden bei 40° C, engt die Lösung ein, filtriert und reinigt den Rückstand über 450 g Kieselgel (Laufmittel Aethylacetat/Hexan 1:1,5). Man erhält 3.4 g Endprodukt, $n_D^{30} =$ 1,5868.

Beispiel 1.7: Herstellung von 1-acetoxy-1-(3-pyridinyl)-2-(2,4-dichlorphenoxy)-butan der Formel

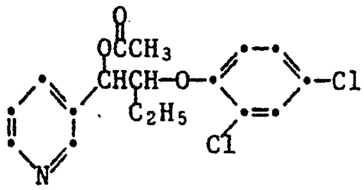

Verb. Nr. 3.1

5,2 g 1-(3-Pyridinyl)-2-(2,4-dichlorphenoxy)-butan-1-ol (Beispiel 1.4) in 25 ml Pyridin werden mit 1,8 ml Acetanhydrid versetzt und bei Raumtemperatur über Nacht gerührt. Die Lösung wird eingedampft, in Ethylacetat aufgenommen, mit Wasser und gesättigter NaCl-Lösung gewaschen, getrocknet ($Na_2SO_4$) und eingedampft. Man erhält 5,0 g Produkt, $n_D^{30} =$ 1,5521.

An Stelle von Acetanhydrid können auch 1,2 ml Acetylchlorid oder Acetylbromid verwendet werden, die in 5 ml Eisessig gelöst zu einer Lösung der gleichen Menge Ausgangsprodukt in 25 ml Eisessig bei ca. 40°C getropft werden. Dann wird das Reaktionsgemisch noch 1 Stunde gerührt und aufgearbeitet.

Auf analoge Weise werden auch folgende Verbindungen der Formel I hergestellt

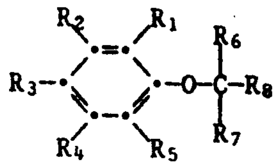

(I)

| Nr. | $R_1$ | $R_2$ | $R_3$ | $R_4$ | $R_5$ | $R_6$ | $R_7$ | $R_8$ | Physikalische Daten |
|---|---|---|---|---|---|---|---|---|---|
| 6 | H | H | Cl | H | H | H | H | 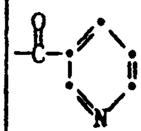 | Smp.:107-108°C |
| 7 | H | H | Br | H | H | H | H | 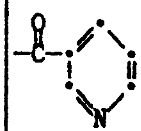 | Smp.:118-119°C |
| 8 | H | H | F | H | H | H | H | 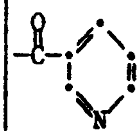 | Smp.:109-111°C |
| 9 | H | H | $CH_3$ | H | H | H | H | 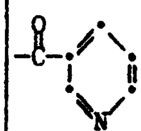 | Smp.: 96-97°C |
| 10 | H | Cl | H | Cl | H | H | H | 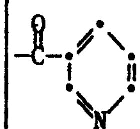 | Smp.:102-104°C |
| 11 | Cl | H | H | H | H | H | H | 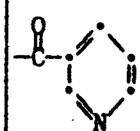 | Smp.: 86-88°C |
| 12 | H | H | $CF_3$ | H | H | H | H | 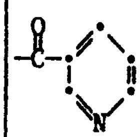 | Smp.:127-129°C |
| 13 | H | Cl | H | H | H | H | H | 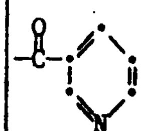 | Smp.: 91-92°C |
| 14 | H | $CF_3$ | H | H | H | H | H | 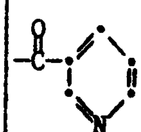 | Smp.: 65-67°C |

Fortsetzung:

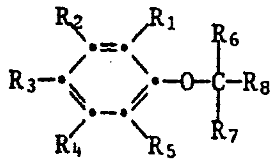

(I)

| Nr. | $R_1$ | $R_2$ | $R_3$ | $R_4$ | $R_5$ | $R_6$ | $R_7$ | $R_8$ | Physikalische Daten |
|---|---|---|---|---|---|---|---|---|---|
| 15 | H | $CH_3$ | Cl | H | H | H | H | 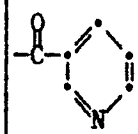 | Smp.: 93-95°C |
| 16 | H | H | $OCH_3$ | H | H | H | H | 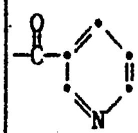 | Smp.: 85-87°C |
| 17 | Cl | H | H | H | H | $CH_3$ | H | 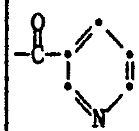 | $n_D^{30°}$ = 1,5821 |
| 18 | H | Cl | H | H | H | $CH_3$ | H | 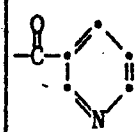 | Smp.: 59-61°C |
| 19 | H | H | Cl | H | H | $CH_3$ | H | 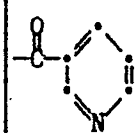 | Smp.: 76-77°C |
| 20 | H | H | $CF_3$ | H | H | $CH_3$ | H | 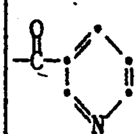 | $n_D^{30°}$ = 1,5220 |
| 21 | H | $CF_3$ | H | H | H | $CH_3$ | H | 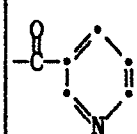 | $n_D^{30°}$ = 1,5212 |
| 22 | H | H | F | H | H | $CH_3$ | H | 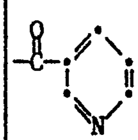 | Smp.: 71-73°C |
| 23 | H | H | Br | H | H | $CH_3$ | H | 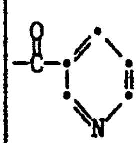 | Smp.: 69-71°C |

Fortsetzung:

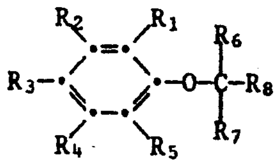

(I)

| Nr. | $R_1$ | $R_2$ | $R_3$ | $R_4$ | $R_5$ | $R_6$ | $R_7$ | $R_8$ | Physikalische Daten |
|---|---|---|---|---|---|---|---|---|---|
| 24 | H | H | $CH_3$ | H | H | $CH_3$ | H | 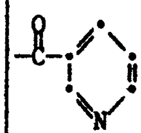 | Smp.: 91-93°C |
| 25 | H | $CH_3$ | Cl | H | H | $CH_3$ | H | 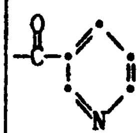 | Smp.: 59-61°C |
| 26 | H | Cl | H | Cl | H | $CH_3$ | H | 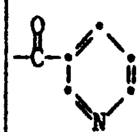 | $n_D^{30°}$ = 1,5869 |
| 27 | Cl | H | Cl | H | H | $C_4H_9$(t) | H | 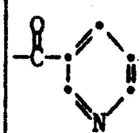 | $n_D^{40°}$ = 1,5601 |
| 28 | H | H | $OCH_3$ | H | H | $CH_3$ | H | 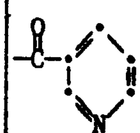 | $n_D^{30°}$ = 1,5701 |
| 29 | Cl | H | Cl | H | H | $C_2H_5$ | H | 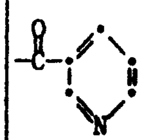 | Sdp.:152-154/ $10^{-2}$ mbar |
| 30 | Cl | H | Cl | H | H | $C_4H_9$(n) | H | 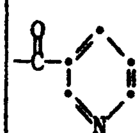 | $n_D^{40°}$ = 1,5600 |
| 31 | Cl | H | Cl | H | H | $C_3H_7$(n) | H | 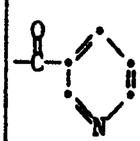 | $n_D^{30°}$ = 1,5662 |
| 32 | H | H | Cl | H | H | $C_2H_5$ | H | 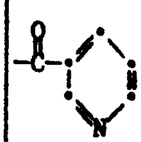 | $n_D^{30°}$ = 1,5755 |

Fortsetzung:

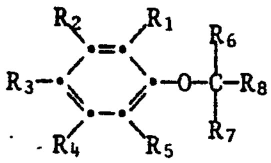

(I)

| Nr. | $R_1$ | $R_2$ | $R_3$ | $R_4$ | $R_5$ | $R_6$ | $R_7$ | $R_8$ | Physikalische Daten |
|---|---|---|---|---|---|---|---|---|---|
| 33 | Cl | H | Cl | H | H | $CH_3$ | $CH_3$ | 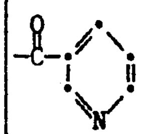 | $n_D^{30°} = 1,5753$ |
| 34 | Cl | H | Cl | H | H | $C_2H_5$ | $CH_3$ | 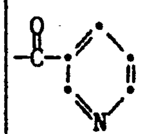 | $n_D^{30°} = 1,5722$ |
| 35 | Cl | H | Cl | H | H | H | H | 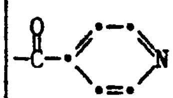 | Smp.:125-126°C |
| 36 | Cl | H | Cl | H | H | $C_2H_5$ | H | 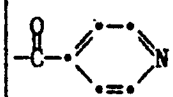 | $n_D^{30°} = 1,5770$ |
| 37 | Cl | H | Cl | H | H | $C_2H_5$ | H | 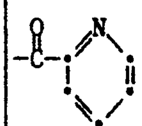 | $n_D^{30°} = 1,5740$ |
| 38 | Cl | H | Cl | H | H | $C_3H_7(n)$ | H | 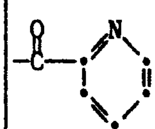 | $n_D^{30°} = 1,5662$ |
| 39 | Cl | H | Cl | H | H | $C_3H_7(n)$ | H | 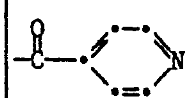 | |
| 40 | Cl | H | Cl | H | H | $C_6H_5$ | H | 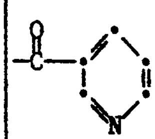 | Smp. 105-107°C |
| 41 | Cl | H | H | H | H | $C_3H_7(i)$ | H | 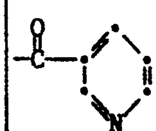 | $n_D^{30°} = 1,5705$ |

Auf gleiche Art oder nach Art einer der weiter oben angegebenen Methoden lassen sich auch folgende Verbindungen der Formel

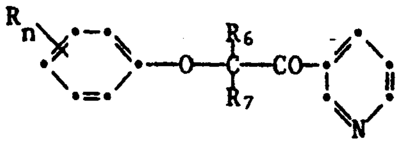 herstellen (n = Index 1, 2 oder 3);

Me = Methyl, Et = Aethyl, nPr = n-Propyl, iPr = Isopropyl

| Verb. Nr. | $R_n$ | $R_6$ | $R_7$ | Physikal. Daten |
|---|---|---|---|---|
| 42 | 4-Cl | iPr | H | |
| 43 | 4-Cl | nPr | H | $n_D^{30}$ = 1,5628 |
| 44 | 4-Cl | n-Butyl | H | $n_D^{40}$ = 1,5519 |
| 45 | 4-Cl | Et | Me | |
| 46 | 2-F, 4-Cl | Et | H | |
| 47 | 2-F, 4-Cl | nPr | H | |
| 48 | 2-F, 4-Cl | iPr | H | |
| 49 | 4-F | Et | H | $n_D^{30}$ = 1,5468 |
| 50 | 4-F | nPr | H | |
| 51 | 4-F | iPr | H | |
| 52 | 4-Br | Et | H | $n_D^{40}$ = 1,5809 |
| 53 | 4-Br | Et | Me | |
| 54 | 4-Br | nPr | H | |
| 55 | 4-Br | iPr | H | |
| 56 | 4-Br | n-Butyl | H | |
| 57 | 4-Phenyl | Et | H | $n_D^{40}$ = 1,6083 |
| 58 | 4-Phenyl | nPr | H | |
| 59 | 4-Phenyl | iPr | H | |
| 60 | 4-$OCH_3$ | nPr | H | |
| 61 | H | Et | H | |
| 62 | H | nPr | H | |
| 63 | 4-tert.Butyl | nPr | H | |
| 64 | 4-Metoxy | Et | H | |
| 65 | 4-Methoy | iPr | H | |
| 66 | 4-$CF_3$ | iPr | H | |

Fortsetzung:

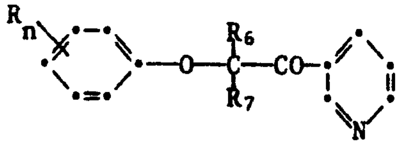

Me = Methyl, Et = Aethyl, nPr = n-Propyl, iPr = Isopropyl

| Verb. Nr. | $R_n$ | $R_6$ | $R_7$ | Physikal. Daten |
|---|---|---|---|---|
| 67 | 3-$CF_3$ | nPr | H | |
| 68 | 4-$COOCH_3$ | Et | H | |
| 69 | 4-$CF_3O$ | nPr | H | |
| 70 | 4-$CF_3O$ | iPr | H | |
| 71 | 4-$CF_3O$ | Et | H | $n_D^{40}$ = 1,5021 |
| 72 | 4-$CHF_2O$ | Et | H | |
| 73 | 4-$CHF_2O$ | nPr | H | |
| 74 | 4-$CHF_2O$ | iPr | H | |
| 75 | 4-CN | Et | H | |
| 76 | 2,4-diCl | Allyl | H | |
| 77 | 2,4-diCl | Propargyl | H | |
| 78 | 3,4-diCl | nPr | H | $n_D^{35}$ = 1,5713 |
| 79 | 2,3-diCl | nPr | H | |
| 80 | 3-Cl | Et | H | |
| 81 | 4-$CHF_2O$ | Me | Me | |
| 82 | 2,4-diCl | Benzyl | H | |
| 83 | 4-Cl | Benzyl | H | |
| 84 | 2-Cl, 4-F | Et | H | $n_D^{40}$ = 1,5514 |
| 85 | 2-Cl, 4-Br | iPr | H | |
| 86 | 2,4-$diF_2$ | n-Butyl | H | |
| 87 | 4-$CF_3$ | Et | H | $n_D^{40}$ = 1,5125 |
| 88 | 2,4-diCl | $C_6H_4Cl(4)$ | H | Smp. 94-96°C |
| 89 | 4-Cl | $C_6H_5$ | H | Smp. 111-113°C |
| 90 | 4-Cl | $C_6H_4Cl(4)$ | H | Smp. 86-88°C |
| 91 | 4-Cl | sec-Butyl | H | $n_D^{35}$ = 1,5559 |

Fortsetzung:

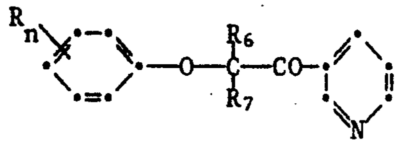

Me = Methyl, Et = Aethyl, nPr = n-Propyl, iPr = Isopropyl

| Verb. Nr. | $R_n$ | $R_6$ | $R_7$ | Physikal. Daten |
|---|---|---|---|---|
| 92 | 2,4-diCl | sec-Butyl | H | $n_D^{35}$ = 1,5652 |
| 93 | 4-Cl | $C_6H_4Cl(2)$ | H | Smp. 95-97°C |
| 94 | 2,4-diCl | $C_6H_4Cl(2)$ | H | hochviskos |
| 95 | 2,4-diCl | $C_6H_3diCl(2,6)$ | H | hochviskos |
| 96 | 2,4,6-triCl | Et | H | $n_D^{35}$ = 1,5710 |
| 97 | $2-CH_3$, 4-Cl | Et | H | $n_D^{35}$ = 1,5623 |

Nach Art der Beispiele 1.4 oder 1.5 oder nach Art einer der weiter oben gegebenen Methoden lassen sich auch folgende Verbindungen der Formel

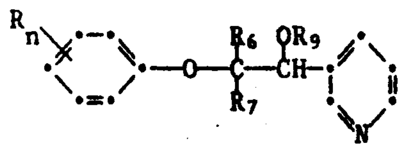

herstellen (n = Index 1, 2 oder 3; Me = Methyl, Et = Aethyl, nPr = n-Propyl, iPr = Isopropyl)

| Verb. Nr. | $R_n$ | $R_6$ | $R_7$ | $R_9$ | Physikal. Daten |
|---|---|---|---|---|---|
| 2.1 | 4-Cl | Et | H | H | |
| 2.2 | 4-Cl | Et | H | Me | |
| 2.3 | 2,4-diCl | Et | H | Allyl | |
| 2.4 | 2,4-diCl | Et | H | Propargyl | |
| 2.5 | 2,4-diCl | nPr | H | H | $n_D^{40}$= 1,5678 |
| 2.6 | 2,4-diCl | iPr | H | H | $n_D^{50}$= 1,5620 |
| 2.7 | 2,4-diCl | n-Pr | H | Me | $n_D^{40}$= 1,5491 |
| 2.8 | 2,4-diCl | iPr | H | Me | $n_D^{30}$= 1,5528 |
| 2.9 | 2,4-diCl | n-Butyl | H | H | $n_D^{40}$= 1,5531 |
| 2.10 | 2,4-diCl | n-Butyl | H | Me | $n_D^{30}$= 1,5470 |
| 2.11 | 2-Cl, 4-F | Et | H | H | $n_D^{40}$= 1,5528 |
| 2.12 | 2-Cl, 4-F | Et | H | Me | |
| 2.13 | 4-F | nPr | H | Me | |
| 2.14 | 4-Br | n-Butyl | H | Me | |
| 2.15 | 2,6-diCl | Me | Me | H | |
| 2.16 | 2,4-diCl | Me | Me | Me | |
| 2.17 | 4-$OCH_3$ | Et | H | H | |
| 2.18 | 4-$CF_3$ | n-Pr | H | H | |
| 2.19 | 4-$CF_3$ | n-Pr | H | Me | |
| 2.20 | 4-$CF_3O$ | Et | H | H | |
| 2.21 | 4-$CF_3O$ | Et | H | Et | |
| 2.22 | 4-CN | Et | H | Me | |
| 2.23 | 2,4-diF | n-Butyl | H | Me | |

Fortsetzung:

| Verb. Nr. | $R_n$ | $R_6$ | $R_7$ | $R_9$ | Physikal. Daten |
|---|---|---|---|---|---|
| 2.24 | 3,4-diCl | Et | H | H | |
| 2.25 | 2,3-diCl | nPr | H | H | |
| 2.26 | 2,3-diCl | nPr | H | Me | |
| 2.27 | 4-Me | Et | H | Me | |
| 2.28 | 2,4-diCl | Phenyl | H | H | |
| 2.29 | 2,4-diCl | Phenyl | H | Me | |
| 2.30 | 2,4-diCl | Me | H | H | Smp. 88-100°C |
| 2.31 | 2,4-diCl | Me | H | Me | $n_D^{31}$ = 1,5642 |
| 2.32 | 2,4-diCl | H | H | H | Smp. 117-119°C |
| 2.33 | 2,4-diCl | H | H | Me | $n_D^{31}$ = 1,5716 |
| 2.34 | 2,4,6-triCl | Et | H | H | Smp. 118-121°C |
| 2.35 | 2-$CH_3$, 4-Cl | Et | H | H | Smp. 69- 72°C |

Nach Art des Beispiels 1.7 werden auch folgende Acylverbindungen hergestellt:

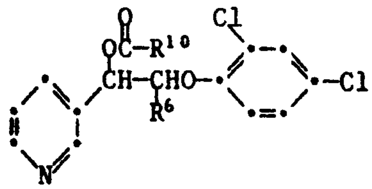

| Verb. Nr. | $R^{10}$ | $R^6$ | Physikalische Konstante |
|---|---|---|---|
| 3.1 | $-CH_3$ | $C_2H_5$ | $n_D^{30} = 1,5521$ |
| 3.2 | $-CH_2CH_3$ | $C_2H_5$ | $n_D^{30} = 1,5470$ |
| 3.3 | $-CH_2CH_2CH_3$ | $C_2H_5$ | $n_D^{30} = 1,5398$ |
| 3.4 | $CF_3$ | $C_2H_5$ | $n_D^{30} = 1,5188$ |
| 3.5 | $-C(CH_3)_3$ | $C_2H_5$ | $n_D^{30} = 1,5350$ |
| 3.6 | 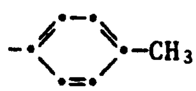 | $C_2H_5$ | $n_D^{30} = 1,5783$ |
| 3.7 | 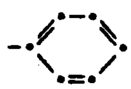 | $C_2H_5$ | $n_D^{30} = 1,5774$ |
| 3.8 | 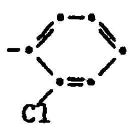 | $C_2H_5$ | $n_D^{30} = 1,5831$ |
| 3.9 | 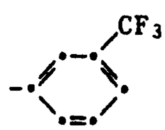 | $C_2H_5$ | $n_D^{30} = 1,5408$ |
| 3.10 | 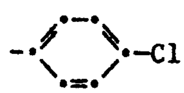 | $C_2H_5$ | $n_D^{35} = 1,5824$ |
| 3.11 | 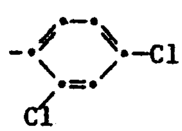 | $C_2H_5$ | $n_D^{35} = 1,5882$ |
| 3.12 | 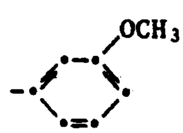 | $C_2H_5$ | $n_D^{30} = 1,5781$ |

Fortsetzung Tabelle 3

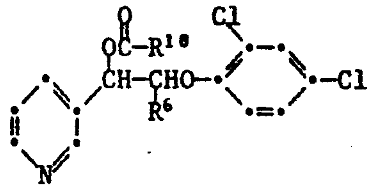

| Verb. Nr. | $R^{10}$ | $R^6$ | Physikalische Konstante |
|---|---|---|---|
| 3.13 | $CH_3$ | $C_4H_9(n)$ | $n_D^{30}$ = 1,5395 |
| 3.14 | $CH_2OCH_3$ | $C_2H_5$ | $n_D^{30}$ = 1,5471 |
| 3.15 | $CH_2OCH_3$ | $C_4H_9(n)$ | $n_D^{30}$ = 1,5400 |
| 3.16 | 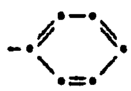 | $C_4H_9(n)$ | $n_D^{30}$ = 1,5702 |
| 3.17 | 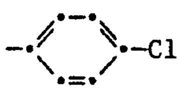 | $C_4H_9(n)$ | $n_D^{30}$ = 1,5739 |
| 3.18 | 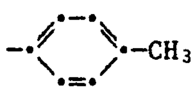 | $C_4H_9(n)$ | $n_D^{30}$ = 1,5673 |
| 3.19 | 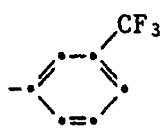 | $C_4H_9(n)$ | $n_D^{30}$ = 1,5396 |

sowie auch folgende Acylverbindungen:

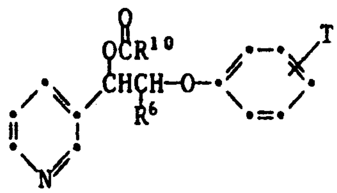

| Verb. Nr. | $R^{10}$ | $R^6$ | T | Physikalische Konstante |
|---|---|---|---|---|
| 4.1 | $-C_3H_7$(iso) | $C_2H_5$ | 2,4-di-Cl | $n_D^{30}$ 1,5394 |
| 4.2 | 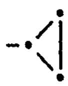 | $C_2H_5$ | 2,4-di-Cl | $n_D^{30}$ 1,5559 |
| 4.3 | $-CH_2CH_2CH_3$ | $C_4H_9$(n) | 2,4-di-Cl | |
| 4.4 | $-CH_2CH_3$ | $C_4H_9$(n) | 2,4-di-Cl | $n_D^{30}$ 1,5388 |
| 4.5 | $-CH_3$ | $C_2H_5$ | 4-Cl | |
| 4.6 | $-CH_3$ | $C_4H_9$(n) | 4-Cl | |
| 4.7 | $CH_3$ | $C_3H_7$(n) | 2,4-di-Cl | |
| 4.8 | $CH_2CH_3$ | $C_3H_7$(n) | 2,4-di-Cl | |
| 4.9 | $CH_2CH_2CH_3$ | $C_3H_7$(n) | 2,4-di-Cl | |
| 4.10 | $C_3H_7$(iso) | $C_3H_7$(n) | 2,4-di-Cl | |
| 4.11 | $-CH_2CH_3$ | $C_3H_7$(n) | 4-Cl | |
| 4.12 | $-CH_2CH_3$ | $C_3H_7$(iso) | 2,4-di-Cl | $n_D^{50}$ 1,5350 |
| 4.13 | $-CH_3$ | $C_3H_7$(iso) | 2,4-di-Cl | Smp. 116-117°C |
| 4.14 | 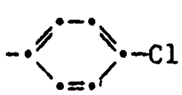 | $C_3H_7$(n) | 2,4-di-Cl | |
| 4.15 | 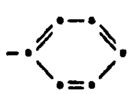 | $C_3H_7$(n) | 2,4-di-Cl | |
| 4.16 | 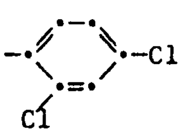 | $C_3H_7$(n) | 2,4-di-Cl | |
| 4.17 | $-C(C_2H_5)=CH_2$ | $C_2H_5$ | 2,4-di-Cl | |
| 4.18 | $-C(CH_3)_3$ | $C_3H_7$iso | 2,4-di-Cl | $n_D^{50}$ 1,5250 |
| 4.19 | $-C_2H_5$ | $C_3H_7$iso | 2,4-di-Cl | |
| 4.20 | $-C_3H_7$iso | $C_3H_7$iso | 2,4-di-Cl | $n_D^{50}$ 1,5294 |
| 4.21 | 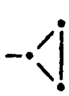 | $C_3H_7$iso | 2,4-di-Cl | $n_D^{50}$ 1,5425 |

Fortsetzung Tabelle 4

| Verb. Nr. | $R^{10}$ | $R^6$ | T | Physikalische Konstante |
|---|---|---|---|---|
| 4.22 | 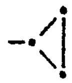 | $C_4H_9n$ | 2,4-di-Cl | $n_D^{30}$ 1,5468 |
| 4.23 | $-C_3H_7iso$ | $C_4H_9n$ | 2,4-di-Cl | $n_D^{30}$ 1,5312 |
| 4.24 | $-C(CH_3)_3$ | $C_4H_9n$ | 2,4-di-Cl | $n_D^{30}$ 1,5284 |
| 4.25 | $CH_3$ | $C_2H_5$ | 2-$CH_3$, 4-Cl | $n_D^{30}$ 1,5484 |
| 4.26 | $CH_3$ | $C_2H_5$ | 2,4,6-tri-Cl | $n_D^{30}$ 1,5603 |
| 4.27 | $CH_3$ | $C_2H_5$ | 2-Cl, 4-F | $n_D^{30}$ 1,5496 |
| 4.28 | $CF_3$ | $C_3H_7iso$ | 2,4-di-Cl | |
| 4.29 | 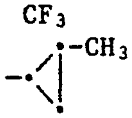 | $C_3H_7iso$ | 2,4-di-Cl | |

Beispiel 2: Formulierungsbeispiele für Wirkstoffe der Formel I gemäss dem Herstellungsbeispiel 1

(% = Gewichtsprozent)

| 2.1 Emulsions-Konzentrate | a) | b) | c) | d) |
|---|---|---|---|---|
| Wirkstoff gemäss den Herstellungsbeispielen oder den Tabellen 1-4 | 10 % | 25 % | 40 % | 50 % |
| Ca-Dodecylbenzolsulfonat | - | 5 % | 8 % | 6 % |
| Ricinusöl-polyäthylenglykoläther (36 Mol AeO) | - | 5 % | - | - |
| Tributylphenol-polyäthylenglykoläther (30 Mol AeO) | - | - | 12 % | 4 % |
| Ricinusölthioxilat | 25 % | - | - | - |
| Cyclohexanon | - | - | 15 % | 20 % |
| Butanol | 15 % | - | - | - |
| Xylolgemisch | - | 65 % | 25 % | 20 % |
| Essigester | 50 % | - | - | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss den Herstellungsbeispielen oder den Tabellen 1-4 | 10 % | 5 % |
| Aethylenglykol-monomethyläther | - | - |
| Polyäthylenglykol (MG 400) | 70 % | - |
| N-Methyl-2-pyrrolidon | 20 % | - |
| Epoxidiertes Kokosnussöl | - | 1 % |
| Benzin (Siedegrenzen 160-190°C) | - | 94 % |

Die Lösungen sind zur Anwendung in Form kleinster Tropfen geeignet.

| 2.3 Spritzpulver | a) | b) | c) |
|---|---|---|---|
| Wirkstoff gemäss den Herstellungsbeispielen oder den Tabellen 1-4 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünne lassen.

| 2.4 Suspensions-Konzentrat | |
|---|---|
| Wirkstoff gemäss den Herstellungsbeispielen oder den Tabellen 1-4 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |

| | |
|---|---|
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3:

Biologische Beispiele:

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

b) Systemische Wirkung

Zu Weizenpflanzen werden 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

Verbindungen aus der Tabelle zeigen gegen Puccinia-Pilze eine sehr gute Wirkung. Unbehandelte aber infizierte Kontrollpflanzen zeigen einen Puccinia-Befall von 100 %. Die Verbindungen 2-7, 19, 29, 30, 2.5-2.8, 2.11, 2.34, 3.1, 3.2, 3.14, 4.1, 4.2, 4.4, 4.22-4.27 hemmen den Puccinia-Befall auf 10 % oder weniger.

Beispiel 3.2: Wirkung gegen Cercospora arachidicola auf Erdnusspflanzen

Residual-protektive Wirkung

10-15 cm hohe Erdnusspflanzen werden mit einer aus Spritzpulver der Wirksubstanz hergestellten Spritzbrühe (0,006 Aktivsubstanz) besprüht und 48 Stunden später mit einer Konidiensuspension des Pilzes infiziert. Die infizierten Pflanzen werden während 72 Stunden bei ca. 21° C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigen Erdnusspflanzen, die mit Wirkstoffen aus der Tabelle behandelt werden, einen stark reduzierten Cercospora-Befall. Die Verbindungen 2, 19, 29, 2.5, 2.6, 2.7, 2.8, 2.11, 3,1. 3.2, 4.1, 4.2, 4.4, 4.25 und andere verhindern das Auftreten von Flecken fast vollständig (0-10 %).

Beispiel 3.3: Wirkung gegen Erysiphae graminis auf Gerste

a) Residual protektive Wirkung

Ca. 8 cm hohe Gerstenpflanzen werden mit einer aus Spritzpulver des Wirkstoffes hergestellten Spritzbrühe (0,002 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22° C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Endvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem

Gewächshaus bei ca. 22°C aufgestellt und der Pilzbefall nach 10 Tagen beurteilt.

Verbindungen der Formel I zeigen gute Wirkung gegen Erysiphe-Pilze. Unbehandelte, aber infizierte Kontrollpflanzen zeigen einen Erysiphe-Befall von 100 %. Die Verbindungen 2, 3, 5, 8, 19, 20, 23, 27, 29, 30, 96, 97, 2.5-2.11, 2.30-2.35, 3.1-3.3, 3.11, 3.13-3.15, 3.19, 4.1, 4.2, 4.4, 4.22-4.27 und andere hemmen den Pilzbefall bei Gerste auf 0 bis 5 %.

Beispiel 3.4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt. Die Verbindungen der Formel I hemmen den Krankheitsbefall auf weniger als 10 %, z.B. Verb. Nr. 29, 30, 3.1, 3.2 und andere. Unbehandelte aber infizierte Kontrolltriebe werden dagegen 100%ig befallen.

Beispiel 3.5: Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21°C erfolgt die Beurteilung des Pilzbefalls. Die Verbindungen aus der Tabelle hemmen in vielen Fällen die Pilzinfektion sehr stark. Bei einer Konzentration von 0,02 % erweisen sich die Verbindungen der Formel I als voll wirksam. Der Krankheitsbefall liegt bei 10 % oder weniger, z.B. nach Behandlung mit den Verbindungen Nr. 3, 5, 11, 29, 30, 2.5-2.8, 3.1, 3.2, 4.1, 4.4, 4.23.

Der Botrytis-Befall unbehandelter aber infizierter Bohnenpflanzen beträgt 100 %.

Beispiel 3.6: Wirkung gegen Piricularia oryzae auf Reispflanzen

Residual-protektive Wirkung

Reispflanzen werden nach zweiwöchigerAnzucht mit einer aus Spritzpulver des Wirkstoffes hergestellten Spritzbrühe (0,002 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall beurteilt.

Reispflanzen, die mit einer Spritzbrühe behandelt worden sind, die als Aktivsubstanz eine der Verbindungen der Formel I enthält, zeigen im Vergleich zu unbehandelten Kontrollpflanzen (100 % Befall) weniger als 20 % Pilzbefall, z.T. keinen Pilzbefall.

**Patentansprüche**

1. Ein Pyridinylderivat der Formel I

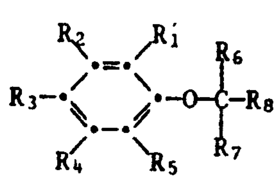

(I),

worin

$R_1$ bis $R_5$ unabhängig voneinander Wasserstoff, Halogen, $C_1$-$C_6$-Alkyl, $C_1$-$C_6$-Halogenalkyl, $C_1$-$C_6$-Alkoxy, $C_1$-$C_6$-Halogenalkoxy, Cyano, $C_1$-$C_6$-Alkoxycarbonyl oder Phenyl,

$R_6$ und $R_7$ unabhängig voneinander Wasserstoff, $C_1$-$C_6$-Alkyl, $C_3$-$C_6$-Alkenyl oder $C_3$-$C_6$-Alkinyl sowie Phenyl oder Benzyl, deren aromatischer Ring unsubstituiert oder durch Halogen, $C_1$-$C_6$-Alkyl, $C_1$-$C_6$-Halogenalkyl, $C_1$-$C_6$-Alkoxy oder $C_1$-$C_6$-Halogenalkoxy substituiert sein kann,

$R_8$ 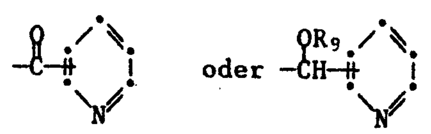 und

$R_9$ Wasserstoff, $C_1$-$C_6$-Alkyl, $C_3$-$C_6$-Alkenyl, $C_3$-$C_6$-Alkinyl oder Benzyl, das unsubstituiert oder im Ring

durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert sein kann, bedeuten, mit der Massgabe, dass eine CO-Gruppe im Substituenten $R_8$ in 3-oder 4-Stellung des Pyridinrings steht, falls $R_1$, $R_2$, $R_4$, $R_5$ und $R_7$ gleichzeitig Wasserstoff, $R_3$ Methoxy und $R_6$ Methyl bedeuten, und wobei ferner

$R_9$ einen Acylrest $R_{10}$-CO- bedeuten kann, in dem $R_{10}$ $C_1$-$C_6$-Alkyl, $C_3$-$C_6$-Alkenyl, $C_3$-$C_6$-Alkinyl, $C_2$-$C_5$-Alkoxyalkyl oder $C_1$-$C_6$-Haloalkyl oder einen $C_3$-$C_6$-Cycloalkylrest bedeutet, der unsubstituiert oder durch $C_1$-$C_3$-Alkyl substituiert sein kann, oder einen der Reste Phenyl, Benzyl, Phenethyl darstellt, die unsubstituiert oder im Ring durch Halogen, $C_1$-$C_6$Alkyl, $C_1$-$C_6$Halogenalkyl, $C_1$-$C_6$Alkoxy, $C_1$-$C_6$Halogenalkoxy substituiert sein können.

2. Eine Verbindung der Formel I gemäss Anspruch 1, worin $R_1$ bis $R_9$ die angegebene Bedeutung haben, mit Ausnahme der Definition $R_9$ = $R_{10}$CO-.

3. Eine Verbindung der Formel I gemäss Anspruch 1, worin

$R_1$ bis $R_5$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, oder einer dieser Substituenten auch Phenyl sein kann

$R_6$ und $R_7$ unabhängig voneinander Wasserstoff, $C_1$-$C_6$-Alkyl, $C_3$-$C_5$-Alkenyl, $C_3$-$C_5$-Alkinyl, Phenyl oder Benzyl bedeuten, die im Ring durch Halogen substituiert sein können, und

$R_9$ Wasserstoff, $C_1$-$C_4$-Alkyl, $C_3$-$C_4$-Alkenyl, $C_3$-$C_4$-Alkinyl, Benzyl oder Halobenzyl darstellt, oder aber die Bedeutung $R_{10}$CO- hat, in der $R_{10}$ $C_1$-$C_4$-Alkyl, $C_3$-$C_4$-Alkenyl, $C_3$-$C_4$-Alkinyl, $C_2$-$C_5$-Alkoxyalkyl, $C_1$-$C_4$-Haloalkyl oder einen $C_3$-$C_6$-Cycloalkylrest bedeutet, der unsubstituiert oder durch $C_1$-$C_3$-Alkyl substituiert ist, oder einen der Reste Phenyl oder Benzyl darstellt, die unsubstituiert oder maximal zweifach durch Halogen, $C_1$-$C_2$-Alkyl, $C_1$-$C_2$-Alkoxy oder $C_1$-$C_2$-Halogenalkyl, das 1-3 Halogenatome enthält, substituiert sein können, während $R_8$ die für Formel I angegebene Bedeutung hat.

4. Eine Verbindung der Formel I gemäss Anspruch 1, worin

$R_1$ bis $R_5$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, oder einer dieser Substituenten auch Phenyl sein kann

$R_6$ und $R_7$ unabhängig voneinander Wasserstoff, $C_1$-$C_6$-Alkyl, $C_3$-$C_5$-Alkenyl, $C_3$-$C_5$-Alkinyl, Phenyl oder Benzyl bedeuten, die im Ring durch Halogen substituiert sein können, und

$R_9$ Wasserstoff, $C_1$-$C_4$-Alkyl, $C_3$-$C_4$-Alkenyl, $C_3$-$C_4$-Alkinyl, Benzyl oder Halobenzyl darstellt, während $R_8$ die für Formel I angegebene Bedeutung hat.

5. Eine Verbindung der Formel I gemäss Anspruch 4, worin

$R_8$ CO-Pyridinyl(3) oder CH($OR_9$)-Pyridinyl(3) darstellt

$R_1$ = Wasserstoff, Methyl, Aethyl oder Halogen,

$R_2$ = Wasserstoff,

$R_3$ = Wasserstoff, Halogen, $C_1$-$C_3$-Halogenalkyl, $C_1$-$C_3$-Halogenalkoxy, CN oder Phenyl,

$R_4$ = Wasserstoff oder Halogen,

$R_5$ = Wasserstoff oder Halogen,

$R_6$ = $C_1$-$C_4$-Alkyl, $C_3$-$C_5$-Alkenyl, $C_3$-$C_5$-Alkinyl, Phenyl oder Benzyl darstellt, die im Ring durch Halogen substituiert sein können,

$R_7$ = Wasserstoff oder $C_1$-$C_3$-Alkyl ist, und

$R_9$ = Wasserstoff, $C_1$-$C_4$-Alkyl, Allyl, Propargyl, Benzyl oder $R_{10}$CO-bedeutet, wobei $R_{10}$ $C_1$-$C_4$-Alkyl, $C_3$-$C_4$-Alkenyl, $C_1$-$C_4$-Haloalkyl oder $C_3$-$C_6$-Cycloalkyl ist, das unsubstituiert oder durch Methyl oder Aethyl substituiert ist.

6. Eine Verbindung der Formel I gemäss Anspruch 5, worin $R_1$ bis $R_8$ die angegebenen Bedeutungen haben, $R_6$ zusätzlich Wasserstoff ist, und

$R_9$ = Wasserstoff, $C_1$-$C_4$-Alkyl, Allyl, Propargyl, oder Benzyl darstellt.

7. Eine Verbindung der Formel I gemäss Anspruch 6, worin

$R_1$ = Wasserstoff, Fluor oder Chlor,

$R_3$ = Fluor, Chlor oder Brom, $CF_3$, $CF_3$-O- oder $CHF_2$-O-, und $R_2$, $R_4$ und $R_5$ Wasserstoff bedeuten, $R_6$ Wasserstoff, $C_2$-$C_4$-Alkyl, Allyl, Propargyl, Phenyl oder Chlorphenyl,

$R_7$ = Wasserstoff oder Methyl, und

$R_9$ = Wasserstoff, Methyl, Aethyl, Isopropyl, allyl oder Benzyl darstellen.

8. Eine Verbindung der Formel I gemäss Anspruch 6, worin

$R_8$ = CO-Pyridinyl(3) oder CH($OR_9$)Pyridinyl(3) darstellt,

$R_1$ = Wasserstoff, Methyl, Fluor oder Chlor,

$R_3$ = Fluor, Chlor oder Brom, $CF_3$, $CF_3$-O- oder $CHF_2$-O-, und $R_2$, $R_4$ und $R_5$ Wasserstoff bedeuten, $R_6$ $C_2$-$C_4$-Alkyl, Allyl, Propargyl, Phenyl oder Chlorphenyl ist,

$R_7$ = Wasserstoff oder Methyl, und

$R_9$ die Gruppe $R_{10}$CO- bedeutet, in der $R_{10}$ für $C_1$-$C_4$-Alkyl, $C_3$-$C_4$-Alkenyl, $C_1$-$C_2$-Haloalkyl oder $C_3$-$C_5$-Cycloalkyl steht, das unsubstituiert oder durch Methyl oder Aethyl substituiert ist.

9. Eine Verbindung der Formel I gemäss Anspruch 7, worin $R_6$ für einen der genannten aliphatischen Substituenten steht.

10. Eine Verbindung der Formel I gemäss Anspruch 1, worin $R_1$ und $R_3$ Chlor und $R_2$, $R_4$, $R_5$ und $R_7$ Wasserstoff bedeuten, $R_6$ $C_1$-$C_4$-Alkyl ist, und $R_8$ CO-Pyridinyl(3) oder CH($OR_9$)-Pyridinyl(3) darstellt, wobei $R_9$ Wasserstoff oder $R_{10}$CO- bedeutet und $R_{10}$ $C_1$-$C_4$-Alkyl, $C_2$-$C_5$-Alkoxyalkyl, $C_3$-$C_4$-Alkenyl, $CF_3$, Cyclopropyl oder Methylcyclopropyl ist.

11. Eine Verbindung der Formel I gemäss Anspruch 10, worin $R_{10}$ Methyl, Aethyl, Propyl, Isopropyl, Cyclopropyl, Butyl, sek. Butyl, Isobutyl oder tert. Butyl darstellt, während $R_1$ bis $R_8$ die angegebenen Bedeutungen haben.

12. 1-(3-Pyridinyl)-2-(2,4-dichlorphenoxy)-butan-1-on der Formel

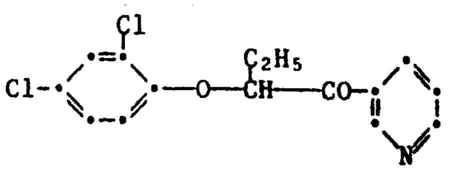

13. Eine Verbindung, ausgewählt aus
1-(3-Pyridinyl)-2-(2,4-dichlorphenoxy)-butan-1-ol oder
1-(3-Pyridinyl)-1-methoxy-2-(2,4-dichlorphenoxy)-butan.

14. Eine Verbindung ausgewählt aus
1-(3-Pyridinyl)-1-acetoxy-2-(2,4-dichlorphenoxy)-butan,
1-(3-Pyridinyl)-1-propionyloxy-2-(2,4-dichlorphenoxy)-butan oder
1-(3-Pyridinyl)-1-methoxyacetoxy-2-(2,4-dichlorphenoxy)-butan.

15. Eine Verbindung ausgewählt aus
1-(3-Pyridinyl)-2-(4-chlorphenoxy)-hexan-1-on,
1-(3-Pyridinyl)-2-(2,4-dichlorphenoxy)-pentan-1-ol oder
1-(3-Pyridinyl)-2-(2,4-dichlorphenoxy)-hexan-1-ol.

16. Verfahren zur Herstellung von Verbindungen gemäss Anspruch 1, dadurch gekennzeichnet, dass man entweder a) einen Ester der Formel II

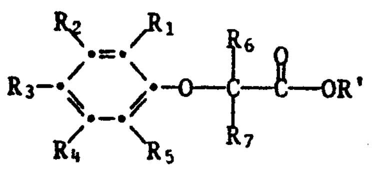 (II)

worin R' für $C_1$-$C_4$-Alkyl, $C_3$-$C_4$-Alkenyl oder für Phenyl oder Benzyl steht, die unsubstituiert oder durch Alkyl, Alkoxy, Halogen, $NO_2$, CN ringsubstituiert sein können, mit einem Halogenid der Formel III

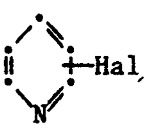 (III)

in Gegenwart eines Metallierungsmittels wie Mg oder wie Butyllithium zur Reaktion bringt oder b) eine Verbindung der Formel IV

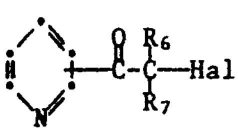 (IV)

mit einem Phenol der Formel V

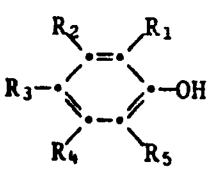 (V)

in Gegenwart einer Base reagieren lässt und die gemäss a) oder b) entstandenen Ketone der Formel I, sofern gewünscht, durch Hydrierung und, sofern gewünscht, anschliessende Reaktion mit einer Verbindung der Formel VII
R''Hal (VII)

in Verbindungen der Formel I überführt
worin $R_8$

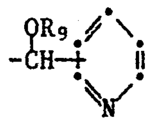

ist und $R_9$ Wasserstoff, $C_1$-$C_6$-Alkyl, $C_3$-$C_6$-Alkenyl, $C_3$-$C_6$-Alkinyl oder Benzyl, das im Ring durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert sein kann, bedeutet; während $R_1$ bis $R_7$ die für die Formel I im Anspruch 1 angegebene Bedeutung haben, worin ferner Hal in den Verbindungen III, IV und VII Halogen bedeutet, und R" für $C_1$-$C_6$-Alkyl, $C_3$-$C_6$-Alkenyl, $C_3$-$C_6$-Alkinyl oder Benzyl steht, das unsubstituiert oder im Ring durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert ist;

oder, sofern gewünscht, dass man die nach der Hydrierung entstandenen Hydroxyl-Verbindungen der Formel I ($R_9$ = Wasserstoff) mit einer entsprechenden Carbonsäure $R_{10}COOH$ oder ihrem Säurehalogenid oder ihrem Säureanhydrid zur Reaktion bringt.

17. Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I gemäss Anspruch 1 enthält, zusammen mit einem geeigneten Trägermaterial.

18. Mittel gemäss Anspruch 17, dadurch gekennzeichnet, dass es als mindestens eine aktive Komponente eine Verbindung der Formel I gemäss Anspruch 2 enthält.

19. Mittel gemäss Anspruch 17, dadurch gekennzeichnet, dass es als mindestens eine aktive Komponente eine Verbindung der Formel I gemäss einem der Ansprüche 3, 5 oder 8 enthält.

20. Mittel gemäss Anspruch 17, dadurch gekennzeichnet, dass es als mindestens eine aktive Komponente eine Verbindung der Formel I gemäss einem der Ansprüche 4, 6, 7, 9, 12 oder 13 enthält.

21. Mittel gemäss Anspruch 17, dadurch gekennzeichnet, dass es als mindestens eine aktive Komponente eine Verbindung der Formel I gemäss einem der Ansprüche 10, 11 oder 14 enthält.

22. Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel I auf die Pflanze oder deren Standort oder auf Pflanzenteile appliziert.

23. Verfahren gemäss Anspruch 22, wobei die Pflanzenteile das Saatgut sind.

Europäisches Patentamt

# EUROPÄISCHER RECHERCHENBERICHT

Nummer der Anmeldung

EP 86810430.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 742 173 (BAYER)<br>* Ansprüche 1-4 *<br>-- | 1,16, 17,22 | C 07 D 213/30<br>C 07 D 213/50<br>A 01 N 43/40 |
| A | EP - A2/A3 - 0 117 485 (HOFFMANN-LA ROCHE)<br>* Ansprüche 1,14,17 *<br>-- | 1,17, 22 | |
| D,A | DE - A1 - 2 909 754 (THOMAE GMBH)<br>* Anspruch 10c; Seite 21; Beispiel Aa) *<br>---- | 1 | |

| RECHERCHIERTE SACHGEBIETE (Int. Cl.4) |
|---|
| C 07 D 213/00 |

Der vorliegende Recherchenbericht wurde für alle Patentansprüche erstellt.

| Recherchenort | Abschlußdatum der Recherche | Prüfer |
|---|---|---|
| WIEN | 07-01-1987 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82